# EUROPEAN PATENT APPLICATION

(11) **EP 4 039 420 A1**
(43) Date of publication of application: **10.08.2022**
(21) Application number: 21305161.8
(22) Date of filing: 05.02.2021
(51) Int. Cl.: B25J 9/16, B25J 19/00, A61B 34/30, A61B 34/00

(54) **BRAKES PILOTING SYSTEM**

(71) Applicant: Ganymed Robotics, 75116 Paris (FR)
(72) Inventor: CARMENT, Thomas, 75015 Paris (FR); BLEUNVEN, Blaise, 75006 Paris (FR); CLAUSE, Thomas, 75019 Paris (FR); CAHEN, Sophie, 75010 Paris (FR)
(74) Representative: Icosa

(57) **Abstract**

The present invention relates to a brake piloting system (10) comprising: a robotic device (12) having at least one movable element (14), at least one brake (16) which, when activated from an open configuration to an activated configuration, enables a deceleration or immobilization of the at least one movable element (14), at least one position sensor (18) aimed at measuring a real time position of the at least one movable element (14) and at least one the microcontroller (22, 24) being configured to activate in real time the at least one brake (16) into a determined configuration.

## Description

### FIELD OF INVENTION

The present invention relates to a brakes piloting system for a robotic device displaying one movable element or a chain of movable elements.

### BACKGROUND OF INVENTION

It is well known in the state of the art, that robotic devices, in particular medical devices, comprising movable elements, have to include position locking systems when the movable element reaches a determined target position, in order to prevent unwanted movement from said movable elements when a target position is reached.

Usually, the control in position as well as the holding at a target position of a movable element is carried out by a motor. However, motors are not always the optimal actuator to lock movable elements into a precise, stable, repeatable position. Motors may then present difficulties to maintain the target position.

To keep the target position of the movable element under a varying external effort or under its own weight, the control in position of motors needs short and high accelerations, resulting in a sudden increase of power consumption as well as the need for complex algorithms.

Further, gear motors provide static or dynamic backlashes, meaning there is an unavoidable and often unpredictable error in the actual position of the moveable element, more precisely on the target position of the moveable element, which leads to reliability issues regarding the reach and the hold of the target position.

Aging is also an issue with gear motors since they comprise a lot of fragile moving parts that are subject to mechanical wear, the combination of wears leading to a decreased precision.

Another drawback of gear motors dimensioned to maintain a strong effort is their important dimensions and weight.

It is thus known from the state of art to use brakes in order to offload the gear motor. In the state of the art, brakes are thus generally used to lock a movable element at a certain position, without controlling the position of movable elements with precision and accuracy, especially during transition phases and once the target position has been reached. For example, in document US 8870141 B2, brakes are used to lock the robotic arm into position when a user decides to lock it, without having a determined target position to reach.

The present invention aims at solving the here-above mentioned issues by using brakes instead of motors to lock robotic movable elements into a determined target position, as well as to control the brakes during transition phases.

### SUMMARY

This invention thus relates to a brake piloting system, said system comprising:
- a robotic device comprising at least one movable element,
- at least one brake which, when activated from an open configuration to an activated configuration, enables a deceleration or immobilization of the at least one movable element,
- at least one position sensor configured to measuring a real time position of the at least one movable element within an internal referential,
- at least one control unit comprising at least one microcontroller storing instructions comprising at least one given target position of the movable element, said at least one microcontroller being connected to the at least one position sensor and to the at least one brake; the microcontroller being configured to:
- receive the real time position of the movable element from the position sensor;
- determine in real time, for each real time position of the movable element, a corresponding configuration of the at least one brake using said real time position of the movable element, a predetermined activation position and the target position; wherein, whenever the movable element is in the target position, the determined configuration is an activated configuration;
- activate in real time the at least one brake into said determined configuration.

The brake control system according to the invention may comprises one or several of the following features, taken in isolation or combined with each other:
According to one embodiment, wherein the control unit comprising at least one mother microcontroller, wherein the instructions are stored, and at least one brake microcontroller connected to the at least one position sensor and to the at least one brake, said at least one brake microcontroller being configured to:
- receive the instructions from the mother microcontroller and the real time position of the movable element from the position sensor,
- determine in real time, for each real time position of the movable element, a corresponding configuration of the at least one brake using said real time position of the movable element, the predetermined activation position and the target position; wherein, whenever the movable element is in the target position, the determined configuration is an activated configuration;
- activate in real time the at least one brake (16) into said determined configuration.

According to one embodiment, the brake microcontroller is further configured to monitor the real time speed of the movable element and further use the real time speed of the movable element to determine the configuration of the at least one brake so as to limit the speed of the movable element to a predetermined maximal speed value.

According to one embodiment, whenever the movable element overcome the predetermined activation position, the predetermined maximal speed value is function of the distance between the real time position of the movable element and the target position of the movable element.

According to one embodiment, the brake microcontroller is further configured to receive from a current sensor a measure in real time of a current applied to the at least one brake and further use the real time current of the movable element to determine the configuration of the at least one brake.

According to one embodiment, the at least one brake is an electromagnetic brake.

According to one embodiment, the at least one brake is a power-off type of brake.

According to one embodiment, the position sensor is an encoder.

According to one embodiment, the robotic device is a medical device.

According to one embodiment, the at least one brake, the at least one position sensor forms an independent functional module.

Another object of the present invention is a brake piloting method, said method being implemented by a system according to any one of the embodiments described above, the method comprising, within the at least one microcontroller of the control unit:
- monitoring the movement of the movable element set into motion by receiving a real time position of the at least one movable element obtained from the at least one position sensor,
- determining a corresponding configuration of the at least one brake, in real time for each real time position of the movable element, using said real time position of the movable element, a predetermined activation position and the target position; wherein, whenever the movable element is in the target position, the determined configuration is an activated configuration,
wherein the microcontroller activates in real time the at least one brake into said determined configuration.

According to one embodiment, the control unit comprises at least one mother microcontroller and at least one brake microcontroller according to any one of the embodiments described above, further comprising:
- sending, by means of the mother microcontroller, at least one target position of the movable element to the at least one brake microcontroller;
wherein monitoring the movement of the movable element and determining a corresponding configuration of the at least one brake, are performed by the at least one brake microcontroller;
and wherein the at least one brake microcontroller activates the at least one brake into the activated configuration.

According to one embodiment, the activation of the at least one brake comprises two phases:
- a transition phase associated to a series of real time determined activation configuration of the at least one brake during which the at least one brake is activated so as to obtain a deceleration of the movable element,
- a locking phase associated to the predetermined target position during which the at least one brake is activated in a way that its activation configuration leads to a locking of the movable element at the target position,
- the transition phase starting when the movable element overcomes the predetermined activation position, the locking phase starting when the movable element reaches the predetermined target position, the locking phase thus chronologically following the transition phase and the locking phase starting when the transition phase ends.

According to one embodiment, the method comprised a third phase whenever the movable element precedes the predetermined activation position, wherein the third phase is a constant speed limitation phase during which the at least one brake microcontroller limits the speed of the movable element to a constant maximum speed value, said speed limitation phase chronologically precedes the transition phase.

According to one embodiment, the at least one brake card monitors the heat emitted by the at least one brake

### BRIEF DESCRIPTION OF THE DRAWINGS

**Figure 1** is a schematized system according to the invention, in which the movable elements are parts of a robotic arm,
**Figure 2** is a schematic view of the electrical architecture of the invention according to a first embodiment,
**Figure 3** is a schematic view of the electrical architecture of the invention according to a second embodiment,
**Figure 4** is an example of a block diagram illustrating the piloting of a brake within its associated brake card.
**Figure 5** is a graph of the speed of the movable element as function of its position according to the embodiment wherein no maximal speed limitation is imposed before reaching the activation position and the deceleration after the activation position is constant.
**Figure 6** is a graph of the speed of the movable element as function of its position according to the embodiment wherein a maximal speed limitation is imposed before reaching the activation position.
**Figure 7** is a graph of the speed of the movable element as function of its position according to the embodiment wherein no maximal speed limitation is imposed before reaching the activation position and the deceleration after the activation position is not constant.
**Figure 8** is a schematic representation of how the information is transmitted between the mother microcontroller, comprising the state machine, the brake microcontroller and the brake.

### DETAILED DESCRIPTION

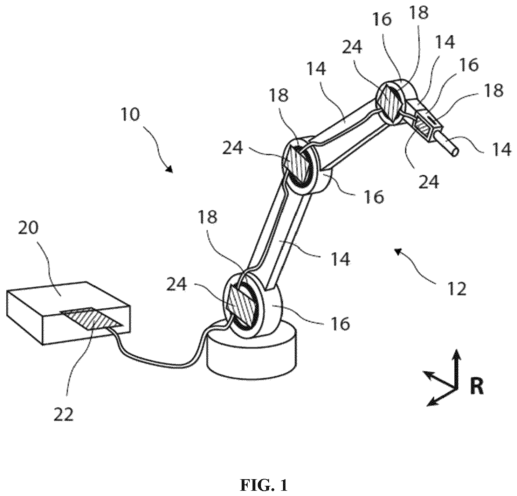

As can be seen on figure 1, a brake piloting system 10 according to the present invention comprises:
- a robotic device 12 comprising at least one movable element 14,
- at least one brake 16 connected to the movable element 14,
- at least one position sensor 18 configured to measure a real time position of each movable element 14 within an internal referential R.

In the embodiment illustrated on figure 1, the robotic device 12 is a robotic arm comprising four movable elements 14 connected in chain. Each movable element 14 is further connected to at least one brake 16 and to at least one position sensor 18. The movable elements 14 can be set into motion, for example manually by means of an operator pulling or pushing them. They might also be set into motion by means of another robotic device, or any external system. The movable elements 14 can be set into motion all together or element by element.

In the current invention, the term "robotic" is understood in its broadest way, including for example cobotic devices, which are robots intended for direct human-robot interaction within a shared space.

Each brake 16 can be activated from an open configuration to an activated configuration. In the present specification the term "configuration" with regards to brakes designates a "state" of the brake:
- in its open configuration, the brake 16 has sensibly no physical interference with the movable element 14 and thus no influence on the movement of the movable element 14 to which it is connected,
- in its activated configurations, the brake 16 exercise a constraint on the movable element 14 leading to a speed limitation and thus enabling a speed limitation to a constant speed value, or a deceleration or an immobilization of the movable element 14 to which the brake is connected.

It is to be noted that each brake 16 only displays one open configuration, but can display a wide range of activated configurations. Each activated configuration displays a determined braking power that is applied to the corresponding movable element 14. A particular active configuration wherein the brake immobilizes the movable element 14 in a specific position is referred to as the locking configuration.

In one embodiment, the brakes 16 can be electromagnetic brakes, more precisely electromagnetic power-off brakes, meaning that the brakes are into their locking configuration when no electrical current flows through them. Advantageously, a permanent magnet inside each brake 16 ensures that it remains in its locking configuration when no current flows through it.

The position sensors 18 connected to each brake 16 and movable element 14 may, for example, be encoders, magnetic or optical, incremental or absolute.

The position of the movable elements 14 can be a relative position, for example a relative angular position of one movable element 14 with regards to another, or a relative linear position of one movable element 14 with regards to another, or an absolute position of the movable element 14 within the internal referential R.

The system 10 further comprises at least one control unit 20 comprising at least one microcontroller (22, 24) configured to store instructions comprising at least one given target position of the movable element 14. A microcontroller contains one or more CPUs (processor cores) along with memory and programmable input/output peripherals. Therefore, the above-mentioned instructions are stored in the memory of the microcontroller.

Said predetermined target position of the movable element 14 is associated to a specific activated configuration of its corresponding brake 16. This specific activated configuration is preferably the locking configuration, wherein the movable element 14 is immobilized at the predetermined target position stored in the microcontroller (22,24). The predetermined target position(s) for each movable element is determined during a preparative stage. In this preparative stage, a planning of the actions is performed by the robotic device and translated into the corresponding target positions in which the robotic device has to be placed in order to accomplish the desired actions.

In the case of application of the system in the medical domain, more precisely for surgery, the predetermined target position is determined during a pre-operative stage. Indeed, for each surgery a preoperative planning is determined, said preoperative planning comprising a list of actions to be performed during the different surgical phases. For example, the actions may comprise the cutting of a portion of a bone and said preoperative planning will comprise the equations of the cutting planes in one referential that may be registered to the internal referential R. Then, one cutting plane may be used to determine the corresponding position of the saw used to cut the bone in the internal referential R, and from this determine the target position of each movable element 14 of the system 10 to reach said cutting plane.

In one embodiment, a predetermined activation position may be either stored into the microcontroller memory or calculated within the microcontroller as a function of the target position, for example.

Said at least one microcontroller (22, 24) is connected to the at least one position sensor 18 and to the at least one brake 16.

In particular the at least one microcontroller (22, 24) receives the real time position of the movable element 14 from the position sensor 18. Then, the microcontroller uses said real time position of the movable element 14, the predetermined activation position and the at least one given target position within the internal referential R to determine in real time a configuration for the at least one brake 16. This step of determining the configuration may be performed for each position of the movable element 14 transmitted by the position sensor (for example, sampling frequency of the sensor). For each iteration, the microcontroller compares the position of the movable element 14 to a predetermined activation position within the internal referential R. Notably, whenever the movable element 14 is in the target position, it sets the determined configuration into an activated configuration. This means that for each position of each movable element 14 within the referential R, the microcontroller determines a determined brake configuration of the corresponding brake 16.

Finally, the microcontroller activates in real time the at least one brake 16 into said determined configuration, which may be an open configuration or activated configuration, notably a locking configuration when the movable element 14 had reached the target position.

In one example, the predetermined activation position and the target position stored are one unique value, corresponding to the target position. In this case the brakes will be always associated to an open configuration (except for the target position) and will be only activated into the locking configuration once the movable element 14 will reach the target position.

According to one embodiment, the control unit 20 comprises a unique microcontroller, for example connected by wires to the brakes 16 and the position sensors 18 (not represented).

In one alternative embodiment, illustrated in figures 1, 2 and 3, the control unit 20 comprises at least one mother microcontroller 22, wherein the instructions are stored, and at least one brake microcontroller 24 and each brake 16 is associated to one brake microcontroller 24. The brake microcontroller 24 is connected to the mother microcontroller 22 and to the position sensor 18 of its corresponding movable element 14. Each movable element 14, its associated brake 16, its associated brake microcontroller 24, and the associated position sensor 18 form thus an independent functional module. This advantageously provides a configuration easy to implement and therefore the system has a mechanical architecture which may be easy to modify and adapt to different circumstances.

The brake microcontroller 24 is configured to receive the instructions from the mother microcontroller 22 and real time information from the position sensor 18. Each brake microcontroller 24 can also send, in real time, feedbacks and information to the mother microcontroller 22.

Each brake microcontroller 24 is further configured to determine in real time, for each received real time position of the movable element 14, a corresponding configuration of the at least one brake 16 using said real time position of the movable element 14, the predetermined activation position and the target position; wherein, whenever the movable element 14 is in the target position, the determined configuration is an activated configuration.

In this embodiment, it is each brake microcontroller 24 that is thus configured to activate each corresponding brake 16 into the determined configuration.

Each brake microcontroller 24 is configured to pilot its corresponding brake(s) 16 whereas mother microcontroller 22 is configured for the global control of the system 10.

In one embodiment, the mother microcontroller 22 is further configured to implement a state machine, which is a mathematical computation model which provides an output based on the actual state of the system and other inputs. The outputs of this state machine may be sent to each brake microcontroller 24 according to the workflow of a preoperative planning and the state of progress of the surgery. These outputs of the state machine may be stored in the mother microcontroller 22 as instruction and comprise the predetermined target position of the movable element 14 of the robotic device 12. The brake microcontroller 24 sends feedback to the mother microcontroller 22 regarding the position of the movable element 14 and the corresponding configuration of its associated brake 16, notably when one target position is reached.

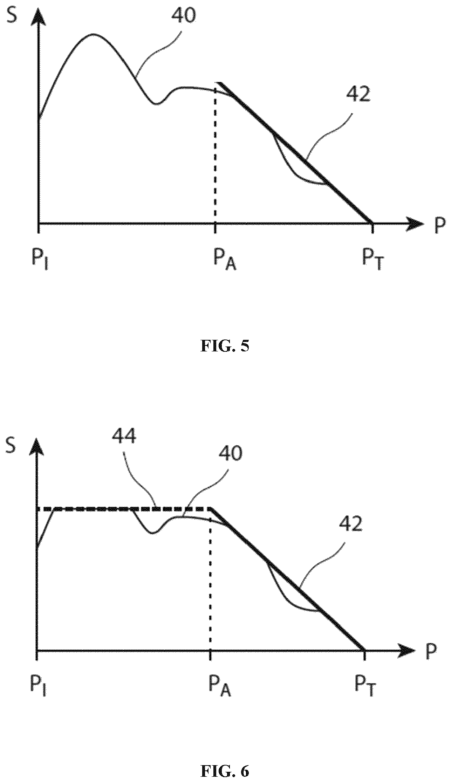

As shown in figures 5 and 6, the real time speed of the movable element 14 may also be taken into account to determine the real time configuration of its associated brake 16. Indeed, the brake microcontroller 24 may be further configured to monitor the real time speed of the movable element 14, using for example an encoder and/or an accelerometer. Then, if the measured speed exceeds a predetermined maximal speed value, the brake microcontroller 24 uses the measured real time speed of the movable element 14 to determine an active configuration of the at least one brake 16 so as to limit the speed of the movable element 14 to the predetermined maximal speed value. If the measured speed does not exceed the predetermined maximal speed value the brake will be in an open configuration.

As shown by the straight line 42 in figures 5 and 6, when the movable element overcomes the predetermined activation position (P_{A}), the predetermined maximal speed value may be function of the distance between the real time position of the movable element 14 and the target position of the movable element (here represented as P_{T}).

More in details, figure 5 shows the case where no speed modulation is performed before that the moveable element reaches the activation position (P_{A}) so that the speed of each movable element 14 will freely depend on the external forces excised on the robotic device 12.

Figure 6 shows a second embodiment where the brake microcontroller 24 is configured to monitor and limit the speed in real time for any position of the movable element 14. In this embodiment, before reaching the activation position (P_{A}), the speed is limited to a constant maximum speed value (straight line 44), meaning as shown by the curve 40, that each movable element 14 cannot exceed a constant speed value. This speed limitation advantageously allows to always maintain the robotic device 12 under control, even if the target position is not close to be reached. Once the movable element overcomes the activation position (P_{A}), the speed is limited as a function of the distance from the target position (P_{T}) so as, to approach the target position will decrease the speed down to zero at the target position. The curve 40 visually shows that the brake microcontroller 24 is configured only to prevent exceeding a predetermined value of speed at a given position by activating the brake into the corresponding activated configurations but lower speed values will not activate the brakes.

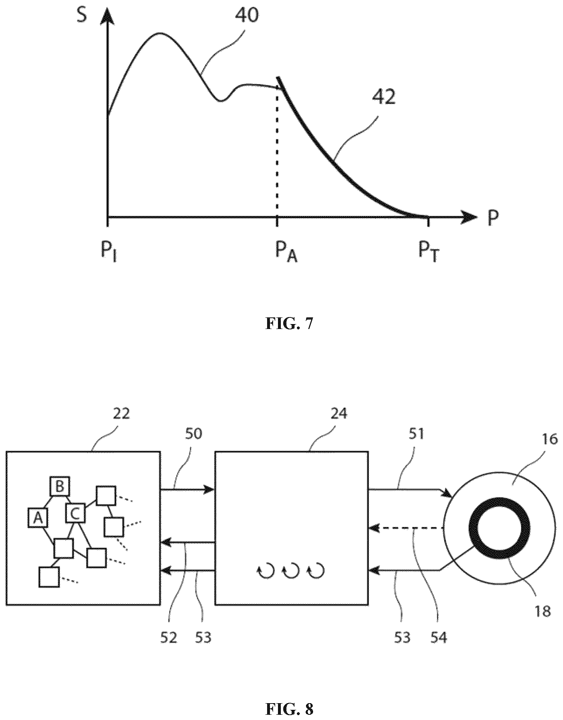

As shown in figure 7, the predetermined maximal speed value may be as well a nonlinear function of the distance between the position of the movable element and the target position.

According to one embodiment, the brake microcontroller 24 is further configured to receive from a current sensor 26 a measure in real time of a current applied to its corresponding brake 16 and further use this measured real time current of the movable element 14 to determine the configuration of the at least one brake 16. This advantageously allows a finer control of the brake.

Figure 8 shows a global view of the information shared between the microcontrollers (22,24), the sensors (position 18, speed and current) and the brake 16. More in details, the mother microcontroller 22, that implement the state machine, sends to the brake microcontroller 24 the instructions 50, the brake microcontroller 24 sends to the brake 16 the piloting voltage 51 to activate the brake in the determined configuration. The sensor 18 sends to the brake microcontroller 24 the measure in real time of the position of the movable element 53 while the current sensor sends to the brake microcontroller 24 the measure in real time of the current 54 flowing in the brake. The brake microcontroller 24 send to the mother microcontroller 22 the information on the position 53, notably when the target position is reached. Other various information 52 may be sent to the mother microcontroller 22 by the brake microcontroller 24.

For most of the positions of each movable element 14, the corresponding configuration of the brake 16 is the open configuration. However, for at least one first real time determined activation position of the movable element 14, the corresponding configuration of the associated brake is an activated configuration. Thus, when the movable element 14 is set into motion and reaches said first real time determined activation position, the associated brake 16 is activated by its corresponding brake microcontroller 24. This brake activation leads the brake 16 to change from its former configuration, the open configuration, to a first determined activated configuration. When the movable element 14 further reaches a second determined activation position, the associated brake 16 is activated by its corresponding brake microcontroller 24 and changes from its former configuration, the first real time determined activated configuration, to a second determined activated configuration. There can be an infinite number of activation positions, depending on the movement imposed to the movable element 14. The activation level of the brake 16 depends on the real time position of the movable element 14. In some embodiments, the activation level of the brake 16 also depends on the real time speed of the movable element 14. Depending on the embodiments, the first determined activation position of the movable element is either speed induced, position induced, or both.

In other words, the piloting of the brake 16 configuration is mainly position driven: when a movable element 14 is set into motion and reaches the predetermined target position within the internal referential R, its corresponding brake 16 is activated by the corresponding brake microcontroller 24, the brake 16 configuration changes into its locking configuration and the movable element 14 is immobilized.

In some embodiments, the piloting of the brake 16 is also speed driven: when a movable element 14 is set into motion at a too high speed with respect to a maximum speed value (which may dependent or not from the predetermined target position), the brake card 24 may also induce a change in the corresponding brake 16 configuration and thus inducing either a deceleration of the movable element 14 or a limitation of the speed of the movable element 14 to a constant speed value. Thus, several types of activation positions can be defined:
- microcontroller one predetermined target position being associated to the locking configuration of the brake 16,
- many real time determined activation positions being associated with active brake configurations which are not the locking configuration.

Independently of any speed limitation, the real time determined activation positions are generally defined spatially, within the internal referential R, surrounding the predetermined target position. This leads each brake 16 to be activated progressively, stepwise or gradually, up to its locking configuration. In other words, this means that, as the movable element 14 is moved from one real time determined activation position to the next one, each brake 16 is activated progressively until the locking configuration is reached. The more a brake 16 is activated, the more intense the deceleration. This progressive activation of the brakes 16 defines a transition phase: this way, the closer the movable element 14 gets to its specific predefined target position, the more difficult it becomes to set said movable element 14 into motion. This transition phase may inform a user that the specific predefined target position of the movable element 14 is soon to be reached.

In some embodiments, each brake microcontroller 24 is able to monitor the heat emitted by its corresponding brake 16, in particular when the brake 16 is maintained in an activated configuration. This advantageously allows to avoid any degradation of the brake and any risk of burning the user.

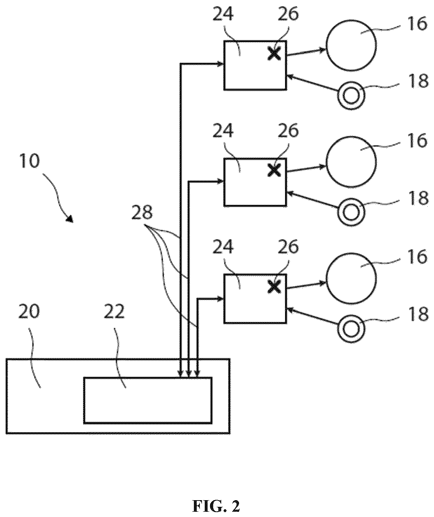

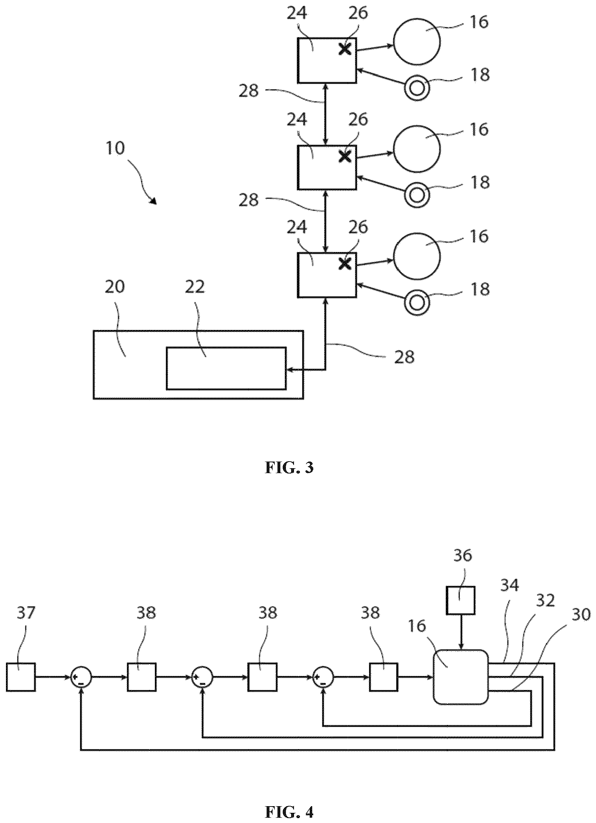

Figures 2 and 3 show two schematic embodiments of the global electronic architecture of the system 10. It can clearly be seen, that each brake microcontroller 24 is connected:
- to at least one brake 16,
- to an encoder 18 which gives the real time position of the movable element 14, and
- to a current sensor 26 which gives the real time amount of electrical current flowing through the brake 16.

A data bus 28 allows the communication between the motherboard 22 and each of the brake microcontroller 24. Figures 2 and 3 show two embodiments of the said data bus 28 connecti ons.

To monitor the position of the movable elements 14 and to monitor and pilot the real time configuration of the brakes 16, each brake microcontroller 24 uses three pieces of information:
- the target position of the movable element 14,
- the real time position of the movable element 14, and
- the current flowing through the brake 16.

As illustrated in the embodiment of figure 4, those pieces of information enable each brake microcontroller 24 to continually apply:
- a current control loop 30 of the at least one brake 16,
- a velocity limitation loop 32 of the movable element 14,
- a position control loop 34 of the movable element 14.

More precisely, the brake microcontroller 24 continually applies a cascade control loop comprising a position control loop, a velocity limitation loop and a current control loop, wherein the current control loop is the inner loop. Advantageously a cascade control loop allows to respond quickly to position, speed and/or current disturbance and so considerably reduce the fluctuations that would have occurred with a single loop control system.

In one embodiment, each control loop 30, 32, 34 is managed by a proportional-integral-derivative corrector (PID) 38. A PID corrector is commonly known in automation and widely used in industrial control systems. A PID corrector continuously applies a correction based on the difference between the desired setpoint and the actual physical value.

The current control loop 30 enables the brake microcontroller 24 to finely control the configuration of its corresponding brake 16 by calculating the voltage to apply at its terminal.

The current control loop optimizes the velocity control by decreasing the response time of the brakes. The current control loop also allows feedback regarding the effort exerted by the brakes 16.

The present invention further relates to a brake piloting method being implemented by a system 10 according to any one of the embodiments hereabove.

The method uses the microcontroller (22,24) of the control unit 20 to perform the following steps:
1. monitoring the movement of the movable element 14 set into motion by receiving a real time position of the at least one movable element 14 obtained from the at least one position sensor 18;
2. determining a corresponding configuration of the at least one brake 16, in real time for each real time position of the movable element 14, using said real time position of the movable element 14 and a predetermined activation position and the target position stored into the microcontroller (24,26); wherein, whenever the movable element 14 is in the target position, the determined configuration is an activated configuration.

The microcontroller (22,24) activates in real time the at least one brake 16 into said determined configuration.

According to the embodiment wherein the control unit 20 comprises at least one mother microcontroller 22 and at least one brake microcontroller 24, the method further comprises sending, by means of the mother microcontroller 22, at least one target position of the movable element 14 to the at least one brake microcontroller 24. In this embodiment, the steps of the method 1 and 2 cited above (i.e. monitoring the movement of the movable element and determining a corresponding configuration of the at least one brake) are performed by the at least one brake microcontroller 24. Finally, it is the at least one brake microcontroller 24 that activates the at least one brake 16 into the activated configuration

As previously mentioned, the activation of the brakes 16 comprises two phases following each other:
- a transition phase during which each brake 16 is activated in a way that its configuration leads to a deceleration of its corresponding movable element 14,
- a locking phase during which each brake 16 is activated in a way that its configuration leads to a locking of the corresponding movable element 14.

The transition phase starts when the movable element 14 overcomes the predetermined activation position and the locking phase starts when the movable element 14 reaches its predetermined target position. The start of the locking phase corresponds to the end of the transition phase.

As already mentioned, in some embodiments, the brake microcontroller 24 monitors the real time speed of the movable element 14 and in those embodiments, each brake 16 is activated proportionally to the speed of its corresponding movable element 14 when the movable element 14 is at the corresponding real time determined activation position. This allows the user of the device 10 to feel that the predetermined target position is soon to be reached and leads thus to a quicker and more precise positioning of the movable element 14.

In the embodiments in which the device 10 includes a maximal allowed speed limit, each brake microcontroller 24 limits the speed of the movable element 14, independently from the position of the movable element 14. In those embodiments, the brake activation comprises a third phase whenever the movable element 14 precedes the predetermined activation position, wherein the third phase is a constant speed limitation phase during which the at least one brake microcontroller 24 limits the speed of the movable element 14 to a constant maximum speed value, said speed limitation phase chronologically precedes the transition phase. This advantageously guarantee a smooth motion so that the user is never surprised by a brutal speed limitation, this feature enables to increase comfort of manipulation of the robotic device, which is particularly interesting in the case of a surgical robotic device during operation.

The present invention is thus about a control in position of one or several brakes connected to movable elements, said movable elements being positioned with precision and accuracy within an internal referential. As the movable elements are displaced by one or several external forces, such as human efforts, the brakes controlled in position aim at reaching a final target configuration and maintaining the movables elements in this position. It allows a fine control of the decelerations and a strong locking at the target position.

## Claims

1. Brake piloting system (10), said system (10) comprising:
- a robotic device (12) comprising at least one movable element (14),
- at least one brake (16) which, when activated from an open configuration to an activated configuration, enables a deceleration or immobilization of the at least one movable element (14),
- at least one position sensor (18) configured to measuring a real time position of the at least one movable element (14) within an internal referential (R),
- at least one control unit (20) comprising at least one microcontroller (22, 24) storing instructions comprising at least one given target position of the movable element (14), said at least one microcontroller (22, 24) being connected to the at least one position sensor (18) and to the at least one brake (16); the microcontroller (22, 24) being configured to:
- receive the real time position of the movable element (14) from the position sensor (18);
- determine in real time, for each real time position of the movable element (14), a corresponding configuration of the at least one brake (16) using said real time position of the movable element (14), a predetermined activation position and the target position; wherein, whenever the movable element (14) is in the target position, the determined configuration is an activated configuration;
- activate in real time the at least one brake (16) into said determined configuration.

2. Brake piloting system (10) according to claim **1,** wherein the control unit comprising at least one mother microcontroller (22), wherein the instructions are stored, and at least one brake microcontroller (24) connected to the at least one position sensor (18) and to the at least one brake (16), said at least one brake microcontroller (24) being configured to:
- receive the instructions from the mother microcontroller (22) and the real time position of the movable element (14) from the position sensor (18)
- determine in real time, for each real time position of the movable element (14), a corresponding configuration of the at least one brake (16) using said real time position of the movable element (14), the predetermined activation position and the target position; wherein, whenever the movable element (14) is in the target position, the determined configuration is an activated configuration;
- activate in real time the at least one brake (16) into said determined configuration.

3. Brake piloting system (10) according to claim **1,** wherein the brake microcontroller (24) is further configured to monitor the real time speed of the movable element (14) and further use the real time speed of the movable element (14) to determine the configuration of the at least one brake (16) so as to limit the speed of the movable element (14) to a predetermined maximal speed value.

4. Brake piloting system (10) according to claim **2,** wherein, whenever the movable element (14) overcome the predetermined activation position, the predetermined maximal speed value is function of the distance between the real time position of the movable element and the target position of the movable element.

5. Brake piloting system (10) according to any one of the preceding claims, wherein the brake microcontroller (24) is further configured to receive from a current sensor (26) a measure in real time of a current applied to the at least one brake (16) and further use the real time current of the movable element (14) to determine the configuration of the at least one brake (16).

6. Brake piloting system (10) according to any one of the preceding claims, wherein the at least one brake (16) is an electromagnetic brake.

7. Brake piloting system (10) according to the preceding claim, wherein the at least one brake (16) is a power-off type of brake.

8. Brake piloting system (10) according to any one of the preceding claims, wherein the position sensor (18) is an encoder.

9. Brake piloting system (10) according to any one of the preceding claims, wherein the robotic device (12) is a medical device.

10. Brake piloting system (10), wherein the at least one movable element (14), the at least one brake (16), the at least one position sensor (18) forms an independent functional module.

11. A brake piloting method, said method being implemented by a system (10) according to any one of claims 1 to 10, the method comprising, within the at least one microcontroller (22,24) of the control unit:
- monitoring the movement of the movable element (14) set into motion by receiving a real time position of the at least one movable element (14) obtained from the at least one position sensor (18),
- determining a corresponding configuration of the at least one brake (16), in real time for each real time position of the movable element (14), using said real time position of the movable element (14), a predetermined activation position and the target position; wherein, whenever the movable element (14) is in the target position, the determined configuration is an activated configuration,
wherein the microcontroller (22,24) activates in real time the at least one brake (16) into said determined configuration.

12. The method of claim **11,** wherein the control unit comprises at least one mother microcontroller (22) and at least one brake microcontroller (24) according to any one of claims **2** to **10,** further comprising:
- sending, by means of the mother microcontroller (22), at least one target position of the movable element (14) to the at least one brake microcontroller (24);
wherein monitoring the movement of the movable element (14) and determining a corresponding configuration of the at least one brake (16), are performed by the at least one brake microcontroller (24);
and wherein the at least one brake microcontroller (24) activates the at least one brake (16) into the activated configuration.

13. Method according to claim **12,** wherein the activation of the at least one brake (16) comprises two phases:
- a transition phase associated to a series of real time determined activation configuration of the at least one brake (16) during which the at least one brake (16) is activated so as to obtain a deceleration of the movable element (14),
- a locking phase associated to the predetermined target position during which the at least one brake (16) is activated in a way that its activation configuration leads to a locking of the movable element (14) at the target position,
the transition phase starting when the movable element (14) overcomes the predetermined activation position, the locking phase starting when the movable element (14) reaches the predetermined target position, the locking phase thus chronologically following the transition phase and the locking phase starting when the transition phase ends.

14. Method according to the preceding claim, wherein the method comprised a third phase whenever the movable element (14) precedes the predetermined activation position, wherein the third phase is a constant speed limitation phase during which the at least one brake microcontroller (24) limits the speed of the movable element (14) to a constant maximum speed value, said speed limitation phase chronologically precedes the transition phase.

15. Method according to any one of claims **9** to **13,** wherein the at least one brake card (24) monitors the heat emitted by the at least one brake (16).
